# EUROPEAN PATENT APPLICATION

(11) **EP 4 043 098 A1**
(43) Date of publication of application: **17.08.2022**
(21) Application number: 20874618.0
(22) Date of filing: 09.10.2020
(51) Int. Cl.: B01F 17/22, C07C 233/49, C11D 1/04, C11D 1/10, A61K 8/44

(54) **SURFACTANT AND COMPOSITION**

(30) Priority: 10.10.2019 JP 2019186919
(71) Applicant: AJINOMOTO CO., INC., Chuo-ku Tokyo 104-8315 (JP)
(72) Inventor: KOSONO, Shuhei, Kawasaki-shi, Kanagawa 210-8681 (JP)
(74) Representative: Strehl Schübel-Hopf & Partner
(86) International application number: PCT/JP2020/038339
(87) International publication number: WO 2021/070941

(57) **Abstract**

The present invention provides a surfactant that has good foaming, foam quality, and solubility even in a weakly acidic region having a pH equivalent to that of the skin, has a low viscosity with high handleability, and can be applied to a product such as a personal care (e.g., a cosmetic). More specifically, the present invention provides a surfactant comprising an N-monounsaturated acyl acidic amino acid or a salt thereof, wherein the unsaturated acyl is an acyl having 10 to 16 carbon atoms.

## Description

### TECHNICAL FIELD

The present invention relates to a surfactant and a composition.

### BACKGROUND ART

A surfactant is used as a personal care, a home care, an agricultural chemical, a paint, an ink, a lubricating oil additive, a cutting oil additive, a rust inhibitor, or the like for various applications such as cleansing, emulsification, solubilization, dispersion, foaming, lubrication, static charge prevention, and rust prevention. For example, in a case of use for a personal care, a surfactant is often blended as a cleanser for the body and hair.

In recent years, a cleanser composition having not only high cleansing power but also little irritation to the skin and excellent feeling of use is desired for the body and hair. In particular, an amino acid type cleanser composition, for example, a cleanser composition containing an N-acylglutamate or an N-acylglycine salt as an active component has attracted attention. A cleanser for the body and hair in a weakly acidic region having a pH equivalent to that of the skin is preferred so as to be milder to the skin. However, in the weakly acidic region, the cleanser cannot maintain transparency or cannot provide sufficient foaming in some cases.

Regarding N-acylglutamic acid which is a kind of N-acyl acidic amino acid, there are reports of studies in which a fatty acid chain length of an acyl group is adjusted to improve a function of N-acylglutamic acid (Patent Literatures 1 and 2). However, these reports only discuss N-acylglutamic acid having an acyl group derived from a fatty acid easily available from natural plants.

### PRIOR ART REFERENCES

### Patent Literature

Patent Literature 1: JP 2009-51945 A
Patent Literature 2: JP 2003-34670 A

### SUMMARY OF INVENTION

### PROBLEM TO BE SOLVED BY THE INVENTION

The object of the present invention is to find a new surfactant by adjusting an acyl group of an N-acyl acidic amino acid, and to solve a problem of an N-saturated acyl acidic amino acid or a salt thereof (a problem such as transparency or foaming in a weakly acidic region) by using the surfactant in addition to an existing N-saturated acyl acidic amino acid or a salt thereof. More specifically, the object of the present invention is to provide a surfactant that has good foaming, foam quality, and solubility even in a weakly acidic region having a pH equivalent to that of the skin, has a low viscosity with high handleability, and hence can be applied to a product such as a personal care (e.g., a cosmetic), and a composition containing the surfactant and having improved oil cleansing power.

### MEANS FOR SOLVING PROBLEM

As a result of intensive studies on the above problems, the present inventors have found that an N-monounsaturated acyl acidic amino acid(s) or a salt(s) thereof wherein the unsaturated acyl is an acyl having 10 to 16 carbon atoms has good foaming, foam quality, and solubility even in a weakly acidic region having a pH equivalent to that of the skin, and has a low viscosity with high handleability, and have completed the present invention. In addition, the present inventors have found that a composition comprising an N-monounsaturated acyl acidic amino acid(s) or a salt(s) thereof wherein the unsaturated acyl is an acyl having 10 to 16 carbon atoms in combination with an N-saturated acyl acidic amino acid(s) or a salt(s) thereof improves oil cleansing power in addition to the effects of the surfactant, and have completed the present invention.

Due to good foaming, foam quality, and solubility even in a weakly acidic region having a pH equivalent to that of the skin, advantages that applicability to the skin is good, precipitation is suppressed, and storage stability is excellent are exhibited. In addition, unlike a general anionic surfactant, an advantage that a low viscosity with high handleability is obtained even at a low pH is also exhibited. A surfactant exhibiting these advantages is considered to be particularly suitable for product applications such as a personal care (e.g., a cosmetic) that can be applied directly to the skin.

In addition, since oil cleansing power is improved, an advantage that more effective cleansing power can be expected is exhibited. Therefore, a composition exhibiting this advantage in addition to the effect of a surfactant is considered to be particularly suitable for a cleanser and the like for the body including the face, hair, and the like among product applications of a personal care.

That is, the present inventors provide the following [1] to [15].
[1] A surfactant comprising an N-monounsaturated acyl acidic amino acid or a salt thereof, wherein the unsaturated acyl is an acyl having 10 to 16 carbon atoms.
[2] The surfactant according to [1], wherein the N-monounsaturated acyl acidic amino acid(s) is a compound represented by the following general formula (1): 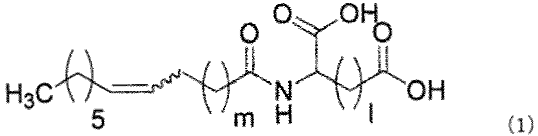 wherein 1 is an integer of 1 or 2, and m is an integer of 0 to 6.
[3] The surfactant according to [1] or [2], wherein a stereochemistry of the N-monounsaturated acyl acidic amino acid is cis.
[4] The surfactant according to any one of [1] to [3], wherein the N-monounsaturated acyl acidic amino acid(s) is N-monounsaturated acylglutamic acid.
[5] A composition comprising:
   component (A) an N-monounsaturated acyl acidic amino acid(s) or a salt(s) thereof, wherein the unsaturated acyl is an acyl having 10 to 16 carbon atoms; and
   component (B) an N-saturated acyl acidic amino acid(s) or a salt(s) thereof.
[6] The composition according to [5], wherein the component (A) comprises:
   (A1) an N-monounsaturated acyl acidic amino acid or a salt thereof, wherein the unsaturated acyl is an acyl having 12 carbon atoms; and
   (A2) an N-monounsaturated acyl acidic amino acid or a salt thereof, wherein the unsaturated acyl is an acyl having 10, 14, or 16 carbon atoms.
[7] The composition according to [5] or [6], wherein the component (B) comprises
   (B1) an N-lauroyl acidic amino acid and/or a salt thereof.
[8] The composition according to [7], wherein the component (B) further comprises
   (B2) an N-saturated acyl acidic amino acid or a salt thereof, wherein the saturated acyl is an acyl having 8, 10, 14, 16 carbon atoms.
[9] The composition according to any of [5] to [8], further comprising component (C) an N-unsaturated fatty acid or a salt thereof.
[10] The composition according to any of [5] to [9], further comprising component (D) a water-soluble medium.
[11] The composition according to any of [5] to [10], wherein a weight ratio of component (A) to a total of component (A) and component (B) (A/(A + B)) is 0.001 to 1.
[12] The composition according to any of [5] to [11], having a pH of 3 to 9.
[13] The composition according to any one of [5] to [12], having a viscosity of 1.5 Pa·s or less.
[14] The composition according to any of [5] to [13], which is an external use composition for skin.
[15] The composition according to any of [5] to [13], which is a cleanser composition.

### EFFECT OF THE INVENTION

The surfactant of the present invention exhibits an effect of having good foaming, foam quality, and solubility even in a weakly acidic region having a pH equivalent to that of the skin, and having a low viscosity with high handleability. Therefore, the surfactant of the present invention is particularly suitable for product applications such as a personal care (e.g., a cosmetic) that can be applied directly to the skin.

The composition of the present invention exhibits an effect of improving oil cleansing power in addition to the above effect. Therefore, the composition of the present invention is particularly suitable for a cleanser and the like for the body including the face, hair, and the like among product applications of a personal care that can be applied directly to the skin.

### EMBODIMENTS FOR CARRYING OUT THE INVENTION

### 1. Surfactant

A surfactant of the present invention comprises an N-monounsaturated acyl acidic amino acid or a salt thereof, wherein the unsaturated acyl is an acyl having 10 to 16 carbon atoms.

Since the acyl moiety of the N-monounsaturated acyl acidic amino acid is a monounsaturated acyl, the viscosity is not extremely reduced. In addition, since the acyl moiety has 10 to 16 carbon atoms, an effect that the viscosity is not excessively increased, a low viscosity with high handleability is obtained, and solubility is good is exhibited. In addition, good foaming and foam quality are obtained.

In the present specification, the "acyl having n carbon atoms" refers to an acyl represented by Cₙ₋₁Hₘ-CO- (a hydrogen atom may be replaced). Here, m is appropriately determined according to the number of carbon atoms and presence or absence of an unsaturated bond.

In the present specification, the "N-monounsaturated acyl acidic amino acid, wherein the acyl has 10 to 16 carbon atoms" refers to a compound represented by Cₙ₋₁Hₘ-CO-NH-CHR-COOH (in which the hydrogen atom represented by Hₘ may be replaced, m is as defined above, and R represents a side chain of an acidic amino acid) as a free form. That is, the "N-monounsaturated acyl acidic amino acid" refers to a compound in which one hydrogen atom on an amino group of an acidic amino acid is replaced with an unsaturated acyl group. The "N-monounsaturated acyl acidic amino acid" may be a free form of an N-monounsaturated acyl acidic amino acid or a salt of the N-monounsaturated acyl acidic amino acid.

Examples of the salt of the N-monounsaturated acyl acidic amino acid include an inorganic salt and an organic salt. Examples of the inorganic salt include a salt of a metal (e.g., a monovalent metal such as lithium, sodium, potassium, rubidium, or cesium, or a divalent metal such as calcium, magnesium, or zinc) and a salt of an inorganic base (e.g., ammonia). Examples of the organic salt include a salt of an organic base (e.g., ethylenediamine, propylenediamine, ethanolamine, monoalkylethanolamine, dialkylethanolamine, diethanolamine, triethanolamine, lysine, arginine, histidine, or ornithine).

In the present specification, the N-monounsaturated acyl acidic amino acid may be indicated as one derived from a fatty acid represented by Cₙ₋₁Hₘ-COOH or a derivative thereof for convenience. In addition, in the present specification, an "unsaturated acyl having n carbon atoms" and a fatty acid and the like from which the unsaturated acyl having n carbon atoms is derived may be represented by "Cn: m". For example, "N-dodecenoyl acidic amino acid (C12: 1)" refers to a compound in which one hydrogen atom on an amino group of an acidic amino acid is replaced with a dodecenoyl group which is an acyl group derived from dodecenoic acid (C12: 1) (C₁₁H₂₁CO-NH-CHR-COOH in which R represents a side chain of the acidic amino acid).

When the N-monounsaturated acyl acidic amino acid is obtained from a monounsaturated fatty acid, the N-monounsaturated acyl acidic amino acid can be obtained, for example, by reacting a monounsaturated fatty acid derivative represented by Cₙ₋₁Hₘ-COX (X represents any monovalent group, for example, a halogen atom selected from the group consisting of fluorine, chlorine, bromine, and iodine) with an acidic amino acid or a salt thereof (examples of the salt include inorganic salts and organic salts as described above).

Examples of the monounsaturated fatty acid from which the N-monounsaturated acyl acidic amino acid is derived include decenoic acid (C10: 1), dodecenoic acid (C12: 1), tetradecenoic acid (C14: 1) (e.g., myristoleic acid), and hexadecenoic acid (C16: 1) (e.g., palmitoleic acid). Dodecenoic acid (C12: 1) or hexadecenoic acid (C16: 1) is preferable, and dodecenoic acid (C12: 1) is more preferable.

The "acidic amino acid" in the "N-monounsaturated acyl acidic amino acid" refers to an amino acid having an acidic side chain. Examples of the acidic amino acid include glutamic acid and aspartic acid, and glutamic acid is preferable.

Examples of the N-monounsaturated acyl acidic amino acid include N-decenoyl acidic amino acid (C10: 1), N-dodecenoyl acidic amino acid (C12: 1), N-tetradecenoyl acidic amino acid (C14: 1), and N-hexadecenoyl acidic amino acid (C16: 1). N-dodecenoyl acidic amino acid (C12: 1) or N-hexadecenoyl acidic amino acid (C16: 1) is preferable. N-dodecenoyl glutamic acid (C12: 1) or N-hexadecenoyl glutamic acid (C16: 1) is more preferable. N-dodecenoylglutamic acid (C12: 1) is still more preferable.

The N-monounsaturated acyl acidic amino acid may comprise one kind of N-monounsaturated acyl acidic amino acid or a salt thereof, or two or more kinds of N-monounsaturated acyl acidic amino acids or salts thereof. That is, the N-monounsaturated acyl acidic amino acid may comprise one or more selected from the group consisting of N-decenoyl acidic amino acid (C10: 1), N-dodecenoyl acidic amino acid (C12: 1), N-tetradecenoyl acidic amino acid (C14: 1), and N-hexadecenoyl acidic amino acid (C16: 1). When two or more kinds of N-monounsaturated acyl acidic amino acids are used, a combination of N-dodecenoyl acidic amino acid (C12: 1) and one or more selected from the group consisting of N-decenoyl acidic amino acid (C10: 1), N-tetradecenoyl acidic amino acid (C14: 1), and N-hexadecenoyl acidic amino acid (C16: 1) is preferable, and a combination of N-dodecenoyl acidic amino acid (C12: 1) and N-hexadecenoyl acidic amino acid (C16: 1) is more preferable from a viewpoint of improving foam quality.

A unsaturated bond site of the N-monounsaturated acyl acidic amino acid is not particularly limited, and the unsaturated bond may be present at a carbon chain terminal of an acyl moiety, between carbon atoms separated by several carbon atoms from the carbon chain terminal of the acyl moiety, or at an α-position of a carbonyl group of the acyl moiety. In particular, the unsaturated bond is preferably present between carbon atoms separated by 6 or 7 carbon atoms from the carbon chain terminal of the acyl moiety. That is, the N-monounsaturated acyl acidic amino acid is preferably a compound represented by the following general formula (1). 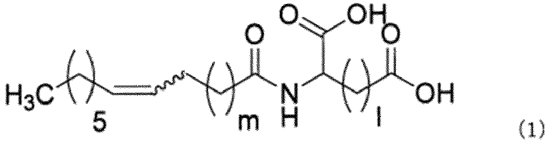 (In general formula (1), 1 is an integer of 1 or 2, and m is an integer of 0 to 6.)

The stereochemistry (unsaturated double bond site) of the N-monounsaturated acyl acidic amino acid may be either cis or trans. However, cis is preferable from a viewpoint of suppressing an increase in viscosity and achieving a low viscosity with high handleability.

### 2. Composition

A composition of the present invention comprises component (A): an N-monounsaturated acyl acidic amino acid(s) or a salt thereof(s), wherein the unsaturated acyl is an acyl having 10 to 16 carbon atoms, and component (B): an N-saturated acyl acidic amino acid(s) or a salt thereof.

The composition of the present invention comprises component (A), and therefore has good foaming, foam quality, and solubility even in a weakly acidic region having a pH equivalent to that of the skin, and has a low viscosity with high handleability. In addition, the composition of the present invention is a composition also comprising component (B) and having variations in an acyl group, and therefore has an effect of improving oil cleansing power.

### (Component (A))

Details of component (A) are as described in the N-monounsaturated acyl acidic amino acid of the surfactant.

### (Component (B))

In the present specification, the "N-saturated acyl acidic amino acid" refers to a compound represented by Cₙ₋₁Hₘ-CO-NH-CHR-COOH (in which the hydrogen atom represented by Hₘ may be replaced, m is as defined above, and R represents a side chain of an acidic amino acid) as a free form. That is, the "N-saturated acyl acidic amino acid" refers to a compound in which one hydrogen atom on an amino group of an acidic amino acid is replaced with a saturated acyl group. The "N-saturated acyl acidic amino acid" may be a free form of an N-saturated acyl acidic amino acid or a salt of the N-saturated acyl acidic amino acid.

Note that the same as the salt of the N-monounsaturated acyl acidic amino acid applies to the salt of the N-saturated acyl acidic amino acid.

In the present specification, the N-saturated acyl acidic amino acid may be indicated as one derived from a fatty acid represented by Cₙ₋₁Hₘ-COOH or a derivative thereof for convenience. In addition, in the present specification, a "saturated acyl having n carbon atoms" and a fatty acid and the like from which the saturated acyl having n carbon atoms is derived may be represented by "Cn". For example, "N-lauroyl acidic amino acid (C12)" refers to a compound in which one hydrogen atom on an amino group of an acidic amino acid is replaced with a lauroyl group which is an acyl group derived from lauric acid (C12) (C₁₁H₂₃CO-NH-CHR-COOH in which R represents a side chain of the acidic amino acid).

When the N-saturated acyl acidic amino acid is obtained from a saturated fatty acid, the N-saturated acyl acidic amino acid can be obtained, for example, by reacting a saturated fatty acid derivative represented by Cₙ₋₁Hₘ-COX (X represents any monovalent group, for example, a halogen atom selected from the group consisting of fluorine, chlorine, bromine, and iodine) with an acidic amino acid or a salt thereof (examples of the salt include inorganic salts and organic salts as described above).

Examples of the N-saturated acyl acidic amino acid include N-capryloyl acidic amino acid (C8), N-caproyl acidic amino acid (C10), N-lauroyl acidic amino acid (C12), N-myristoyl acidic amino acid (C14), N-palmitoyl acidic amino acid (C16), and N-stearoyl acidic amino acid (C18).

The "acidic amino acid" in the "N-saturated acyl acidic amino acid" refers to an amino acid having an acidic side chain. Examples of the acidic amino acid include glutamic acid and aspartic acid, and glutamic acid is preferable.

As the N-saturated acyl acidic amino acid, N-capryloyl acidic amino acid (C8), N-caproyl acidic amino acid (C10), N-lauroyl acidic amino acid (C12), N-myristoyl acidic amino acid (C14), or N-palmitoyl acidic amino acid (C16) is preferable, N-lauroyl acidic amino acid (C12) is more preferable, and N-lauroyl glutamic acid (C12) is still more preferable.

Component (B) may comprise one kind of N-saturated acyl acidic amino acid or a salt thereof, or two or more kinds of N-saturated acyl acidic amino acids or salts thereof. That is, component (B) may comprise one or more selected from the group consisting of N-capryloyl acidic amino acid (C8), N-caproyl acidic amino acid (C10), N-lauroyl acidic amino acid (C12), N-myristoyl acidic amino acid (C14), N-palmitoyl acidic amino acid (C16), and N-stearoyl acidic amino acid (C18). When component (B) comprises two or more kinds of N-saturated acyl acidic amino acids, component (B) preferably comprises N-lauroyl acidic amino acid (C12), and more preferably comprises N-lauroyl acidic amino acid (C12) and one or more selected from the group consisting of N-capryloyl acidic amino acid (C8), N-caproyl acidic amino acid (C10), N-myristoyl acidic amino acid (C14), and N-palmitoyl acidic amino acid (C16) in combination.

When component (B) comprises N-lauroyl acidic amino acid (C12), the content of N-lauroyl acidic amino acid (C12) in component (B) is, for example, 20% by mass or more, preferably 30% by mass or more, more preferably 35% by mass or more, still more preferably 40% by mass or more, and further still more preferably 50% by mass or more. More specifically, the content of N-lauroyl acidic amino acid in component (B) is, for example, 20 to 100% by mass, preferably 30 to 100% by mass, more preferably 35 to 100% by mass, still more preferably 40 to 100% by mass, and further still more preferably 50 to 100% by mass.

A mass ratio of component (A) to the total of component (A) and component (B) (A/(A + B)) is usually 0.001 or more, preferably 0.002 or more, more preferably 0.003 or more, and still more preferably 0.004 or more from a viewpoint of improving solubility at a low pH by component (A), and the like. The mass ratio of component (A) to the total of component (A) and component (B) (A/(A + B)) can be set to 1 or less, preferably less than 1, more preferably 0.8 or less, still more preferably 0.6 or less, and further still more preferably 0.5 or less from a viewpoint of contribution to oil cleansing power due to a large content of component (B), and the like. More specifically, the mass ratio of component (A) to the total of component (A) and component (B) (A/(A + B)) is usually 0.001 to 1, preferably 0.001 or more and less than 1, more preferably 0.002 to 0.8, still more preferably 0.003 to 0.6, and further still more preferably 0.004 to 0.5.

The composition of the present invention may further comprise component (C): an N-unsaturated fatty acid or a salt thereof.

When the composition of the present invention comprises component (C), the foam quality is better, and the oil cleansing power is further improved.

### (Component (C))

The number of carbon atoms of the unsaturated fatty acid is preferably 6 to 22, and more preferably 8 to 18. Examples of the unsaturated fatty acid include hexenoic acid (C6: 1), octenoic acid (C8: 1), decenoic acid (C10: 1), dodecenoic acid (C12: 1), tetradecenoic acid (C14: 1) (e.g., myristoleic acid), hexadecenoic acid (C16: 1) (e.g., palmitoleic acid), octadecenoic acid (C18: 1) (e.g., oleic acid), icosenoic acid (C20: 1) (e.g., eicosenoic acid), and docosenoic acid (C22: 1), and dodecenoic acid (C12: 1) is preferable.

Examples of the salt of the unsaturated fatty acid include an inorganic salt such as a sodium salt, a potassium salt, a calcium salt, a magnesium salt, or an aluminum salt; an organic amine salt such as an ammonium salt, a monoethanolamine salt, a diethanolamine salt, or a triethanolamine salt; and an organic salt such as a basic amino acid salt including an arginine salt and a lysine salt. Among these salts, a triethanolamine salt, a sodium salt, and a potassium salt are preferable.

Component (C) may comprise one kind of unsaturated fatty acid or a salt thereof, or two or more kinds of unsaturated fatty acids or salts thereof. That is, component (C) may comprise one or more selected from the group consisting of hexenoic acid (C6: 1), octenoic acid (C8: 1), decenoic acid (C10: 1), dodecenoic acid (C12: 1), tetradecenoic acid (C14: 1) (e.g., myristoleic acid), hexadecenoic acid (C16: 1) (e.g., palmitoleic acid), octadecenoic acid (C18: 1) (e.g., oleic acid), icosenoic acid (C20: 1) (e.g., eicosenoic acid), and docosenoic acid (C22: 1). When component (C) comprises two or more kinds of unsaturated fatty acids or salts thereof, component (C) preferably comprises dodecenoic acid (C12: 1), and more preferably comprises dodecenoic acid (C12: 1) and one or more selected from the group consisting of octenoic acid (C8: 1), decenoic acid (C10: 1), tetradecenoic acid (C14: 1), and hexadecenoic acid (C16: 1) in combination.

When component (C) comprises dodecenoic acid (C12: 1), the content of dodecenoic acid (C12: 1) in component (C) is, for example, 30% by mass or more, preferably 40% by mass or more, more preferably 50% by mass or more, still more preferably 60% by mass or more, further still more preferably 70% by mass or more, and particularly preferably 80% by mass or more. More specifically, the content of dodecenoic acid (C12: 1) in component (C) is, for example, 30 to 100% by mass, preferably 40 to 100% by mass, more preferably 50 to 100% by mass, still more preferably 60 to 100% by mass, further still more preferably 70 to 100% by mass, and particularly preferably 80 to 100% by mass.

A mass ratio of component (C) to the total of component (A) and component (B) (C/(A + B)) may be 0 or more. When the mass ratio of component (C) to the total of component (A) and component (B) (C/(A + B)) is 0, component (C) is not comprised. The mass ratio can be 0.001 or more, and is preferably 0.01 or more, more preferably 0.1 or more, still more preferably 0.2 or more, further still more preferably 0.4 or more, and particularly preferably 0.5 or more from a viewpoint of contribution of component (C) to improvement of foam quality (density), and the like. The mass ratio of component (C) to the total of component (A) and component (B) (C/(A + B)) is usually 20 or less, preferably 15 or less, more preferably 13 or less, still more preferably 11 or less, and further still more preferably 10 or less from a viewpoint of contribution of an effect of component (A) and component (B), and the like. More specifically, the mass ratio of component (C) to the total of component (A) and component (B) (C/(A + B)) is usually 0.001 to 20, preferably 0.01 to 20, more preferably 0.1 to 15, still more preferably 0.2 to 13, further still more preferably 0.4 to 11, and particularly preferably 0.5 to 10.

The composition of the present invention may comprise component (A), component (B), and an optional component (C) in (D): a water-soluble medium. As the water-soluble medium, any water-soluble solvent can be used. Examples of the water-soluble medium include an aqueous solution. The aqueous solution may have buffering ability or does not have to have buffering ability. Examples of the aqueous solution include water (e.g., distilled water, sterilized distilled water, purified water, physiological saline, or tap water such as city water), a phosphoric acid buffer, a Tris-hydrochloric acid buffer, a TE (Tris-EDTA) buffer, a carbonic acid buffer, a boric acid buffer, a tartaric acid buffer, a glycine buffer, a citric acid buffer, and an acetic acid buffer.

The content of component (A) varies depending on various conditions such as the kinds and concentrations of other components comprised in the composition of the present invention and a pH, and therefore is not particularly limited. When the composition of the present invention is in a form of an aqueous solution, the content of component (A) is, for example, 0.001% by mass or more, preferably 0.002% by mass or more, and more preferably 0.003% by mass or more from a viewpoint of improving solubility at a low pH, and the like. The content of component (A) can be, for example, 60% by mass or less, and is preferably 40% by mass or less. More specifically, the content of component (A) is, for example, 0.001 to 60% by mass, preferably 0.001 to 40% by mass, more preferably 0.002 to 40% by mass, and still more preferably 0.003 to 40% by mass.

The content of component (B) varies depending on various conditions such as the kinds and concentrations of other components comprised in the composition of the present invention and a pH, and therefore is not particularly limited. When the composition of the present invention is in a form of an aqueous solution, the content of component (B) is, for example, 0.01% by mass or more, preferably 0.02% by mass or more, and more preferably 0.03% by mass or more from a viewpoint of improving solubility at a low pH, and the like. The content of component (B) can be, for example, 60% by mass or less, is preferably 40% by mass or less, and is more preferably 10% by mass or less. More specifically, the content of component (B) is, for example, 0.01 to 60% by mass, preferably 0.02 to 40% by mass, and more preferably 0.03 to 10% by mass.

The content of component (C) varies depending on various conditions such as the kinds and concentrations of other components comprised in the composition of the present invention and a pH, and therefore is not particularly limited, but may be 0% by mass or more. When the content of component (C) is 0% by mass, component (C) is not comprised. When the composition of the present invention is in a form of an aqueous solution, the content of component (C) is, for example, 0.001% by mass or more, preferably 0.002% by mass or more, and more preferably 0.003% by mass or more from a viewpoint of contribution to improvement of foam quality (density), and the like. The content of component (C) can be, for example, 5% by mass or less, is preferably 3% by mass or less, and is more preferably 1% by mass or less from a viewpoint of inhibiting the effect of the present invention when the component (C) is comprised in a large amount, and the like. More specifically, the content of component (C) is, for example, 0.001 to 5% by mass, preferably 0.002 to 3% by mass, and more preferably 0.003 to 1% by mass.

The composition of the present invention is preferably weakly acidic from a viewpoint of storage stability due to suppression of growth of various bacteria (antiseptic effect) and low irritation to the skin due to a pH equivalent to that of the skin (weakly acidic). The pH of the composition of the present invention is, for example, 3 to 9 and can be 3 to 8 or 3 to 7. An upper limit of the pH is preferably 8 or less, and more preferably 7 or less. In addition, a lower limit is preferably 4 or more, and more preferably 4.5 or more. The pH of the composition of the present invention is preferably 4 to 8 and more preferably 4 to 7, or preferably 4.5 to 8 and more preferably 4.5 to 7 from a viewpoint of achieving a pH equivalent to that of the skin. The composition of the present invention has excellent solubility even at a low pH, and therefore suppresses precipitation. It can be said that the composition of the present invention has excellent storage stability also in this respect. Furthermore, in general, when the pH of an anionic surfactant decreases, foaming tends to decrease. However, the composition of the present invention has good foaming even at a low pH. The pH can be adjusted using a pH adjusting agent. Examples of the pH adjusting agent include an aqueous solution (buffer) as described above, an acidic substance (e.g., hydrochloric acid, sulfuric acid, nitric acid, or citric acid), and an alkaline substance (e.g., a hydroxide of an alkali metal including sodium and potassium or an alkaline earth metal including calcium).

The composition of the present invention preferably has a low viscosity from a viewpoint of handleability. The viscosity of the composition of the present invention is, for example, 1.5 Pa·s or less, preferably 1 Pa·s or less, more preferably 0.8 Pa·s or less, still more preferably 0.7 Pa·s or less, and further still more preferably 0.5 Pa·s or less. The composition of the present invention has excellent handleability, and therefore can be said to be useful as a cosmetic as a personal care.

In the present specification, the viscosity is a value obtained by measuring an aqueous solution of a composition having a concentration of 10% by mass with a B-type viscometer (DVB-10: B-type viscometer manufactured by Toyo Seiki Seisaku-sho, Ltd., rotor Nos. 20 to 23, 6 to 30 rpm, 25°C, after 30 seconds).

The composition of the present invention may also comprise another component such as an additional cleansing component, a polyhydric alcohol, a thickener, a stabilizer, a preservative, a fragrance, or a pigment. Specific kinds and amounts of these components can be appropriately set.

Examples of the additional cleansing component include a surfactant such as an anionic surfactant, an amphoteric surfactant, or a nonionic surfactant, and a microsolid (e.g., a microsphere or a scrub).

The anionic surfactant comprises one or more anionic groups. Examples of the anionic group include a carboxyl group, a sulfonic acid group, a sulfuric acid group, and a phosphoric acid group. Examples of the anionic surfactant include a higher fatty acid, an N-acylamino acid, an N-acyltaurine, an alkyl ether carboxylic acid, an alkyl phosphoric acid, a polyoxyethylene alkyl ether phosphoric acid, an alkyl sulfuric acid, a polyoxyethylene alkyl ether sulfuric acid, a sulfonic acid compound having an alkyl chain, and salts thereof.

The amphoteric surfactant comprises one or more anionic groups as described above and one or more cationic groups. Examples of the cationic group include an ammonium group, a primary amino group, a secondary amino group, a tertiary amino group, and a quaternary amino group. Examples of the amphoteric surfactant include an amide betaine amphoteric surfactant, a betaine acetate amphoteric surfactant, a sulfobetaine amphoteric surfactant, and an imidazoline amphoteric surfactant (e.g., lauroamphoacetic acid or a salt thereof).

Examples of the nonionic surfactant include an ester type surfactant such as a glycerin fatty acid ester, a sorbitan fatty acid ester, or a sucrose fatty acid ester, an ether type surfactant such as an alkyl polyethylene glycol or a polyoxyethylene alkyl phenyl ether, and a nonionic surfactant such as an alkyl glycoside in which a saccharide and a higher alcohol are bonded to each other by a glycosidic bond or an alkyl polyglycoside.

Examples of the polyhydric alcohol include a dihydric alcohol (e.g., ethylene glycol, propylene glycol, 1,3-propanediol, 1,2-butylene glycol, 1,3-butylene glycol, 2,3-butylene glycol, 1,4-butanediol, 2-butene-1,4-diol, 1,5-pentanediol, 1,2-pentanediol, isoprene glycol, hexylene glycol, diethylene glycol, dipropylene glycol, or monoglyceride (monoacylglycerol)), a trihydric alcohol (e.g., glycerin, trimethylolpropane, or 1,2,6-hexanetriol), a tetrahydric alcohol (e.g., diglycerin or pentaerythritol), an alcohol having a higher valence, and salts thereof (e.g., inorganic salts and organic salts as described above). Examples of the alcohol having a higher valence include a sugar alcohol optionally having a substituent (e.g., a monosaccharide alcohol such as sorbitol, mannitol, sucrose, glucose, or mannose, a disaccharide alcohol such as trehalose, and a polysaccharide alcohol such as hyaluronic acid or xanthan gum), a polymer of a dihydric to tetrahydric alcohol as described above (e.g., polyglycol or polyglycerin), and salts thereof (e.g., inorganic salts and organic salts as described above). The polyhydric alcohol is preferably a dihydric to tetrahydric alcohol, and more preferably a dihydric or trihydric alcohol.

Examples of the thickener include carrageenan, dextrin, methyl cellulose, ethyl cellulose, hydroxyethyl cellulose, hydroxypropyl cellulose, hydroxypropyl methyl cellulose, polyacrylic acid, polymethacrylic acid, carboxyvinyl polymer (carbomer), (acrylic acid/alkyl acrylate (C10-30)) copolymer, and xanthan gum.

Examples of the stabilizer include ascorbic acid, sodium pyrosulfite, and EDTA.

Examples of the preservative include ethyl parahydroxybenzoate, sodium benzoate, salicylic acid, sorbic acid, paraben (methylparaben, propylparaben, or the like), and sodium bisulfite.

Examples of the fragrance include a natural fragrance and a synthetic fragrance. Examples of the natural fragrance include rose oil, jasmine oil, neroli oil, lavender oil, ylang-ylang oil, tubellows oil, clary sage oil, clove oil, peppermint oil, geranium oil, patulie oil, sandalwood oil, cinnamon oil, coriander oil, nutmeg oil, pepper oil, lemon oil, orange oil, bergamot oil, opoponax oil, vetiver oil, oris oil, and oak moss oil. Examples of the synthetic fragrance include limonene (orange), β-caryophyllene (woody), cis-3-hexenol (young green leaves), linalool (lily of the valley), farnesol (floral with fresh green notes), β-phenylethyl alcohol (rose), 2,6-nonadienal (violet or cucumber), citral (lemon), α-hexyl cinnamic aldehyde (jasmine), β-ionone (violet when being diluted), ι-carboxylic (spearmint), cyclopentadecanone (musk), linalyl acetate (bergamot or lavender), benzyl benzoate (balsam), γ-undecalactone (peach), eugenol (clove), rose oxide (green floral), indole (jasmine when being diluted), phenylacetaldehyde dimethyl acetal (hyacinth), auranthiol (orange flower), and menthol (peppermint) (the word in the parentheses indicates an aroma).

Examples of the pigment include an organic pigment (e.g., a red pigment such as Red No. 201, a blue pigment such as Blue No. 404, an orange pigment such as Orange No. 203, a yellow pigment such as Yellow No. 205, a green pigment such as Green No. 3, an organic lake pigments such as zirconium lake, or a natural pigment such as chlorophyll) and an inorganic pigment (e.g., a white pigment such as titanium oxide, a colored pigment such as iron oxide, an extender pigment such as talc, or a pearl pigment such as mica).

The composition of the present invention can be provided in various forms such as powder, liquid, gel, paste, cream, and foam. Note that the composition according to the present invention can be produced by a usual method.

The composition of the present invention can be a cosmetic in any form applicable to, for example, the skin, hair, or scalp according to a usual method. The cosmetic is suitable for applications such as a body shampoo, a hand soap, a facial cleanser, a cleansing lotion, a cleansing cream, a massage cream, and a hair shampoo as a cleanser for animals such as humans. Preferred characteristics of the cosmetic (e.g., pH) are similar to those of the composition of the present invention described above.

In addition, the composition of the present invention can also be used as an additive such as an excipient.

### EXAMPLES

Hereinafter, the present invention will be described in detail with reference to Examples. The following Examples are intended to suitably describe the present invention, and do not limit the present invention.

### Synthesis Example 1: (Production Example of N-dodecenoylglutamic acid (C12: 1))

3.0 g of cis-5-dodecenoic acid was dissolved in 30 ml of methylene chloride and ice-cooled. Three drops of N,N-dimethylformamide (DMF) and 2.0 ml of oxalyl chloride were added thereto, and the resulting mixture was stirred at room temperature for two hours. Methylene chloride in the reaction solution was distilled off by concentration under reduced pressure, and 25 ml of methylene chloride was added thereto again. The resulting mixture was concentrated under reduced pressure. This operation was repeated three times to obtain cis-5-dodecenoic acid chloride. 30 ml of a 1 M sodium hydroxide aqueous solution and 15 ml of acetone were added to and dissolved in 2.4 g of L-glutamic acid, and the resulting mixture was ice-cooled. The previously obtained acid chloride was dissolved in 15 ml of acetone, and the resulting solution was slowly dropped to the glutamic acid aqueous solution. Meanwhile, a 1 M sodium hydroxide aqueous solution was added thereto to adjust the pH to 10 to 11. After the pH did not change, 15 ml of hexane was added thereto, and the reaction solution was cleansed and separated to obtain an aqueous layer. 100 mL of methylene chloride was added thereto, the pH was adjusted to 2 or less with sulfuric acid, and extraction was performed. The methylene chloride layer was dried over anhydrous magnesium sulfate. Methylene chloride was distilled off by concentration under reduced pressure, and 25 ml of methylene chloride was added to the residue again. The resulting mixture was concentrated under reduced pressure. This operation was repeated two times, and 50 ml of methylene chloride was added to the residue and heated and dissolved. After the dissolution, the solution was ice-cooled and crystallized. The precipitated solid was separated and dried under reduced pressure at 40°C to obtain 3.4 g of a target product. It was confirmed by HPLC that a reaction occurred sufficiently.

### Synthesis Example 2: (Production Example of N-hexadecenoyl glutamic acid (C16: 1))

Preparation was performed in a similar manner to the above using 1.7 g of cis-9-hexadecenoic acid to obtain 1.4 g of a target product. It was confirmed by HPLC that a reaction occurred sufficiently.

### Synthesis Example 3: (Production Example of sodium N-saturated C8 to C16 acylglutamate)

Each fatty acid chloride having a corresponding fatty chain length and a glutamic acid salt in a reaction amount were sufficiently reacted to each other under basic conditions. After completion of the reaction, an acid was added thereto for crystallization, and then filtration, a neutralization step, and a drying step were performed to obtain powdery sodium N-saturated acylglutamate. Note that it was confirmed by HPLC that the obtained sodium N-saturated acyl glutamate was sufficiently reacted.

### (Evaluation of foaming, foam quality, solution state, and viscosity)

A 0.3% by mass aqueous solution and a 10% by mass aqueous solution concentration having compositions in Tables 1 to 3 were prepared using a sodium hydroxide aqueous solution and a citric acid aqueous solution.

[Measurement of foaming]: 20 g of a 0.3% by mass aqueous solution having the composition in Tables 1 to 3 was weighed in a 100 mL glass vial container, and the temperature was raised to 35°C. Then, using a vortex (Scientific Industries, VORTEX-GENIE2, 3000 rpm), the height of foam immediately after shaking for 10 seconds was measured as foaming, and evaluation was performed according to the following evaluation criteria.

### (Evaluation criteria)

A: The height of foam is 35 mm or more.
B: The height of foam is 30 mm or more and less than 35 mm.
C: The height of foam is less than 30 mm.

[Evaluation of foam quality]: 20 g of a 0.3% by mass aqueous solution having the composition in Tables 1 to 3 was weighed in a 100 mL glass vial container, and the temperature was raised to 35°C. Then, using a vortex (Scientific Industries, VORTEX-GENIE2, 3000 rpm), foam quality immediately after shaking for 10 seconds was evaluated by five expert panelists according to the following evaluation criteria.

### (Evaluation criteria)

5 points: It is felt that foam has a thickness.
4 points: Rather, it is felt that foam has a thickness.
3 points: It is felt neither that foam has a thickness nor that foam has no thickness.
2 points: Rather, it is felt that foam has no thickness.
1 point: It is felt that foam has no thickness.

The scores of the five expert panelists were summed up, and the foam quality was evaluated based on the following evaluation criteria.

### (Evaluation criteria)

AA: The total score of five panelists is 23 points or more.
A: The total score of five panelists is 20 points or more and less than 23 points.
B: The total score of five panelists is 10 points or more and less than 20 points.
C: The total score of five panelists is less than 10 points.

[Solution state]: In a 30 mL glass vial container, 10 g of a 10% by mass aqueous solution having the composition in Tables 1 to 3 was weighed. Then, the sample was stored at 25°C, and the state of precipitation of crystals after one week was visually confirmed and evaluated according to the following evaluation criteria.

### (Evaluation criteria)

A: The solution is clear.
B: Precipitation is observed at a bottom of the glass vial, or cloudiness is observed in the entire solution.
C: Precipitation is observed in the entire glass vial.

[Viscosity]: In a 30 mL glass vial container, 10 g of a 10% by mass aqueous solution having the composition in Tables 1 to 3 was weighed. Then, the sample was stored at 25°C, and the viscosity at 25°C after one week was visually confirmed by five expert panelists and evaluated according to the following evaluation criteria.

### (Evaluation criteria)

5 points: The viscosity is lower than that of tap water.
4 points: The viscosity is rather lower than that of tap water.
3 points: The viscosity is about the same as that of tap water.
2 points: The viscosity is rather higher than that of tap water.
1 point: The viscosity is higher than that of tap water.

The scores of the five expert panelists were summed up, and the viscosity was evaluated based on the following evaluation criteria.
A: The total score of five panelists is 19 points or more.
B: The total score of five panelists is 10 points or more and less than 19 points.
C: The total score of five panelists is less than 10 points.

[Overall evaluation]: For the above four tests, as evaluation criteria, 5 points, 4 points, and 1 point were set for A, B, and C, respectively. Overall evaluation was performed based on the following evaluation criteria.

### (Evaluation criteria)

A: 18 points or more
B: 16 points or more and 17 points or less
C: 15 points or less

**Table 1**

| | Example | | | | Comparative Example | | | |
|---|---|---|---|---|---|---|---|---|
| | 1 | 2 | 3 | 4 | 1 | 2 | 3 | 4 |
| (A) C12:1Glu | 100 | 100 | 100 | 100 | 0 | 0 | 0 | 0 |
| (B) C12Glu Na | 0 | 0 | 0 | 0 | 100 | 100 | 100 | 100 |
| Total | 100 | 100 | 100 | 100 | 100 | 100 | 100 | 100 |
| pH | 3.0 | 4.0 | 4.5 | 5.5 | 4.5 | 5.5 | 6.0 | 7.0 |
| Foaming | B | B | B | B | A | A | A | C |
| Foam quality | A | A | B | B | A | A | A | C |
| Solution state | A | A | A | A | C | C | C | A |
| Viscosity | B | B | A | A | C | C | C | A |
| Overall | A | A | A | A | C | C | C | C |

As can be seen from the above results, the surfactant of the present invention had good foaming, foam quality, and solubility even in a weakly acidic region having a pH equivalent to that of the skin, and had a low viscosity with high handleability. On the other hand, sodium N-lauroyl glutamate (C12GluNa) had good foaming and foam quality at a low pH, but had poor solubility and handleability (Comparative Example 1 to 3). In addition, at neutrality, sodium N-lauroyl glutamate (C12GluNa) had good solubility and handleability, but had poor foaming and foam quality (Comparative Example 4).

Note that in Table 1, "C12:1Glu" represents N-dodecenoyl glutamic acid, and "C12GluNa" represents sodium N-lauroyl glutamate.

**Table 2**

| | Example | | | | |
|---|---|---|---|---|---|
| | 5 | 6 | 7 | 8 | 9 |
| (A) C12:1Glu | 10 | 30 | 50 | 70 | 90 |
| (B) C12Glu Na | 90 | 70 | 50 | 30 | 10 |
| Total | 100 | 100 | 100 | 100 | 100 |
| pH | 5.5 | 5.5 | 5.5 | 5.5 | 5.5 |
| Foaming | A | A | B | B | B |
| Foam quality | A | A | A | A | A |
| Solution state | B | B | A | A | A |
| Viscosity | A | A | A | A | A |
| Overall | A | A | A | A | A |

As can be seen from the above results, it was indicated that when N-dodecenoyl glutamic acid was used in combination with sodium N-lauroyl glutamate (C12GluNa), the problem of sodium N-lauroyl glutamate (C12GluNa) alone was solved. That is, the composition of the present invention had good foaming, foam quality, and solubility even in a weakly acidic region having a pH equivalent to that of the skin, and had a low viscosity with high handleability.

Note that in Table 2, "C12:1Glu" represents N-dodecenoyl glutamic acid, and "C12GluNa" represents sodium N-lauroyl glutamate.

**Table 3**

| | Example | | | | | | | Comparative Example | |
|---|---|---|---|---|---|---|---|---|---|
| | 10 | 11 | 12 | 13 | 14 | 15 | 16 | 5 | 6 |
| (A) C12:1Glu | 50 | 30 | 10 | 1 | 15 | 30 | 30 | | |
| (A) C16:1Glu | | | | | 15 | | | | |
| (B) C8Glu Na | 3 | 3 | 3 | 3 | 3 | 3 | 3 | 3 | 3 |
| (B) C10Glu Na | 4 | 4 | 4 | 4 | 4 | 4 | 4 | 4 | 4 |
| (B) C12Glu Na | 18 | 38 | 58 | 67 | 38 | 37 | 38 | 58 | 68 |
| (B) C14Glu Na | 16 | 16 | 16 | 16 | 16 | 16 | 16 | 16 | 16 |
| (B) C16Glu Na | 9 | 9 | 9 | 9 | 9 | 9 | 9 | 9 | 9 |
| (C) C12:1-COOH | | | | | | 1 | | | |
| C18:1Glu | | | | | | | | 10 | |
| Total | 100 | 100 | 100 | 100 | 100 | 100 | 100 | 100 | 100 |
| pH | 5.5 | 5.5 | 5.5 | 5.5 | 5.5 | 5.5 | 7.0 | 5.5 | 5.5 |
| Foaming | A | A | A | A | A | A | A | B | C |
| Foam quality | A | A | A | A | AA | AA | A | B | B |
| Solution state | A | A | A | A | A | A | A | C | A |
| Viscosity | A | A | A | A | A | A | A | A | A |
| Overall | A | A | A | A | A | A | A | C | B |

As can be seen from the above results, the composition of the present invention had good foaming, foam quality, and solubility even in a weakly acidic region having a pH equivalent to that of the skin, and had a low viscosity with high handleability.

A case where two or more kinds of N-saturated acyl acidic amino acids were used in combination was superior to Examples described in Table 2 using only one kind of N-saturated acyl acidic amino acid. Further, when two kinds of N-monounsaturated acyl acidic amino acids were used, the foam quality was evaluated as "AA", which is a more favorable result. On the other hand, those using no N-monounsaturated acyl acidic amino acid were poor in overall evaluation.

Note that in Table 3, "C12:1Glu" represents N-dodecenoyl glutamic acid, "C16:1Glu" represents N-hexadecenoyl glutamic acid, "C18:1Glu" represents N-oleoyl glutamic acid, "C8GluNa" represents sodium N-capryloyl glutamate, "C10GluNa" represents sodium N-capryloyl glutamate, "C12GluNa" represents sodium N-lauroyl glutamate, "C14GluNa" represents sodium N-myristoyl glutamate, and "C16GluNa" represents sodium N-palmitoyl glutamate.

The compositions of Examples 11 and 15 and Comparative Example 6 were subjected to the following evaluation test of oil cleansing power. Results thereof are indicated in Table 4.

[Oil cleansing power]: Cleansing power to oil (petrolatum, Sunwhite P-200, Nikko Rica Corporation) applied onto artificial leather was evaluated. The oil cleansing power was evaluated by the following method.

First, artificial leather was weighed with a precision balance, and 0.10 g of petrolatum was applied onto the artificial leather. 0.30 g of each sample was dropped from above the applied petrolatum, and the sample was blended with the petrolatum for 20 seconds in a circular manner with a finger wearing a rubber glove. Thereafter, the artificial leather was put in a 500 mL beaker containing 300 mL of tap water, and stirred at a rotation speed of 100 rpm for 30 seconds to rinse the oil. Finally, the artificial leather was dried in a room controlled in temperature and humidity for three days, the weight thereof was measured, and oil cleansing power was calculated from the following formula (1). The oil cleansing power was evaluated according to the following criteria, and the results thereof are indicated in Table 4.

Formula (1): cleansing ratio = ((weight of petrolatum cleansed off)/(weight of applied petrolatum)) × 100

### (Evaluation criteria)

A: The cleansing ratio is 23% or more.
B: The cleansing ratio is 17.5% or more and less than 23%.
C: The cleansing ratio is less than 17.5%.

**Table 4**

| | Example | | Comparative Example |
|---|---|---|---|
| | 11 | 15 | 6 |
| Oil cleansing power | B | A | C |

As can be seen from the above results, the oil cleansing power of the composition of the present invention was evaluated as "A" or "B". In particular, when the composition comprises an N-monounsaturated fatty acid, a particularly good result with an evaluation of "A" was obtained. On the other hand, the oil cleansing power of the composition of Comparative Example was evaluated as "C", and the composition of Comparative Example was hardly blended with the oil.

The compositions of Examples 17 and 18 and Comparative Example 7 were subjected to the following viscosity measurement.

[Viscosity]: A B-type viscometer manufactured by Toyo Seiki Seisaku-sho, Ltd. was used for viscosity measurement. An aqueous solution diluted to a concentration of 10% was filled in a 50 mL glass vial container, and a viscosity thereof was measured. The measurement was performed under measurement conditions of rotor No: 20 or 21, rotation speed: 12 or 30 rpm, and measurement time: 30 seconds, and results thereof are indicated together in Table 5.

Note that the measurement was also attempted for the 10 % by mass aqueous solution of Comparative Example 1, but precipitation was observed. Therefore, the measurement could not be performed with a viscometer.

**Table 5**

| | Example | | Comparative Example |
|---|---|---|---|
| | 17 | 18 | 7 |
| (A) C12:1Glu | 100 | 30 | 0 |
| (A) C16:1Glu | 0 | 0 | 0 |
| (B) C8Glu Na | 0 | 3 | 3 |
| (B) C10Glu Na | 0 | 4 | 4 |
| (B) C12Glu Na | 0 | 38 | 38 |
| (B) C14Glu Na | 0 | 16 | 16 |
| (B) C16Glu Na | 0 | 9 | 9 |
| C18:1Glu | 0 | 0 | 30 |
| Total | 100 | 100 | 100 |
| pH | 4.5 | 4.5 | 4.5 |
| Viscosity (mPa·s) | 23 | 12 | 1992 |

As can be seen from the above results, each of the compositions of Examples had a low viscosity of 23 or 12 mPa·s as a measured value of viscosity. On the other hand, the composition of Comparative Example had a high viscosity of 1992 mPa·s (1.992 mPa·s) as a measured value of viscosity.

Note that in Table 5, "C12:1Glu" represents N-dodecenoyl glutamic acid, "C16:1Glu" represents N-hexadecenoyl glutamic acid, "C18:1Glu" represents N-oleoyl glutamic acid, "C8GluNa" represents sodium N-capryloyl glutamate, "C10GluNa" represents sodium N-capryloyl glutamate, "C12GluNa" represents sodium N-lauroyl glutamate, "C14GluNa" represents sodium N-myristoyl glutamate, and "C16GluNa" represents sodium N-palmitoyl glutamate.

## Claims

1. A surfactant comprising an N-monounsaturated acyl acidic amino acid or a salt thereof, wherein the unsaturated acyl is an acyl having 10 to 16 carbon atoms.

2. The surfactant according to claim 1, wherein the N-monounsaturated acyl acidic amino acid is a compound represented by the following general formula (1): 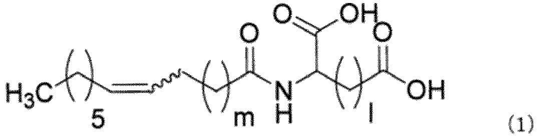 wherein 1 is an integer of 1 or 2, and m is an integer of 0 to 6.

3. The surfactant according to claim 1 or 2, wherein a stereochemistry of the N-monounsaturated acyl acidic amino acid is cis.

4. The surfactant according to any one of claims 1 to 3, wherein the N-monounsaturated acyl acidic amino acid is N-monounsaturated acylglutamic acid.

5. A composition comprising:
component (A) an N-monounsaturated acyl acidic amino acid(s) or a salt(s) thereof, wherein the unsaturated acyl is an acyl having 10 to 16 carbon atoms; and
component (B) an N-saturated acyl acidic amino acid(s) or a salt(s) thereof.

6. The composition according to claim 5, wherein the component (A) comprises:
(A1) an N-monounsaturated acyl acidic amino acid or a salt thereof, wherein the unsaturated acyl is an acyl having 12 carbon atoms; and
(A2) an N-monounsaturated acyl acidic amino acid or a salt thereof, wherein the unsaturated acyl is an acyl having 10, 14, or 16 carbon atoms.

7. The composition according to claim 5 or 6, wherein the component (B) comprises
(B1) an N-lauroyl acidic amino acid and/or a salt thereof.

8. The composition according to claim 7, wherein the component (B) further comprises
(B2) an N-saturated acyl acidic amino acid or a salt thereof, wherein the saturated acyl is an acyl having 8, 10, 14, 16 carbon atoms.

9. The composition according to any one of claims 5 to 8, further comprising component (C) an N-unsaturated fatty acid or a salt thereof.

10. The composition according to any one of claims 5 to 9, further comprising component (D) a water-soluble medium.

11. The composition according to any one of claims 5 to 10, wherein a weight ratio of component (A) to a total of component (A) and component (B) (A/(A + B)) is 0.001 to 1.

12. The composition according to any one of claims 5 to 11, having a pH of 3 to 9.

13. The composition according to any one of claims 5 to 12, having a viscosity of 1.5 Pa·s or less.

14. The composition according to any one of claims 5 to 13, which is an external use composition for skin.

15. The composition according to any one of claims 5 to 13, which is a cleanser composition.
